# EUROPÄISCHE PATENTSCHRIFT

(11) **EP 1 224 924 B1**
(45) Veröffentlichungstag und Bekanntmachung des Hinweises auf die Patenterteilung: **14.05.2008**
(21) Anmeldenummer: 01128907.1
(22) Anmeldetag: 05.12.2001
(51) Int. Cl.: A61K 6/00, A61K 9/00, A61K 36/00

(54) **Mischung zur Behandlung entzündlicher Zahnerkrankungen und zur Verwendung als Wurzelkanalfüllmaterial**
Mixture for the treatment of inflammatory dental diseases and for use as a dental canal filler
Préparation pour le traitement de maladies dentaires inflammatoires et pour l'obturation de canaux dentaires

(30) Priorität: 19.01.2001 DE 10102372
(43) Veröffentlichungstag der Anmeldung: 24.07.2002
(73) Patentinhaber: Merz Pharma GmbH & Co. KGaA, 60318 Frankfurt (DE)
(72) Erfinder: Mordhorst, Wolfgang, 24106 Kiel (DE); Dziuron, Peter, Dr., 24327 Blekendorf (DE)
(74) Vertreter: Glawe, Delfs, Moll

(56) Entgegenhaltungen:
- DE-A- 2 629 694
- DE-A- 3 934 008
- DE-A- 19 744 621
- DE-A- 19 848 597
- US-A- 5 154 613
- DATABASE WPI Section Ch, Week 198119 Derwent Publications Ltd., London, GB; Class A96, AN 1981-33159D XP002276785 & JP 56 026808 A (MITSUBISHI MINING & CEMENT CO), 16. März 1981 (1981-03-16)

## Beschreibung

Die Erfindung betrifft einen Kit, der eine Ölmischung (Komponente a)) und einen Feststoff (Komponente b)) enthält. Gegenstand der Erfindung ist ferner die Verwendung dieses Kits zum Ansetzen einer pastösen Mischung sowie deren Verwendung als Medikament, insbesondere als Suspension zur Behandlung entzündlicher Zahnbett- oder Zahnfleischerkrankungen und als Paste für medizinische Wurzelkanaleinlagen wie auch als Wurzelkanalfüllmaterial.

Aus DE 42 40 713 C1 ist es bekannt, daß eine Mischung aus Calciumhydroxid und Rinderklauenöl bei Knochentraumen die Kollagenneubildung in vivo unterstützen kann. Ebenfalls bekannt ist die Verwendung solcher Mischungen als Wurzelkanalfüllmaterial (DE 29 32 738 C2).

DE-A-197 44 621 offenbart eine Mischung, die enthält a) Paraffine nach DAB und/oder synthetische Wachse ausgewählt aus der Gruppe bestehend aus Montanwachsen, Wachsen aus Erdöl, Fischer-Tropsch-Wachsen, Polyolefinwachsen, Vaseline, Wachsalkoholen, und Oxidaten der vorgenannten Stoffe; b) wenigstens ein Metallhydroxid, wobei die Mischung kein Rinderklauenöl enthält, zur Verwendung als Wundverband. Pflanzliche Öle werden in DE-A-197 44 621 als fakultative Komponenten der vorgenannten Mischung offenbart.

Die Verwendung von Produkten bovinen Ursprungs wird von Teilen der Weltbevölkerung aus religiösen Gründen abgelehnt, bovine Proteine können zudem Allergien verursachen. Aufgrund der Verbreitung von BSE wächst auch in westlichen Kulturen die Skepsis gegenüber der Verwendung von Produkten von bovinen Ursprungs.

Der Erfindung liegt daher die Aufgabe zugrunde, einen Kit der eingangs genannten Art zu schaffen, dessen Bestandteile nicht tierischen, insbesondere nicht bovinen Ursprungs sind und aus dem sich eine als Wurzelkanalfüllmaterial verwendbare Paste sowie eine zum Einbringen in Zahnfleischtaschen geeignete (vorzugsweise dünnflüssige) Suspension ansetzen läßt.

Die Erfindung löst diese Aufgabe durch einen Kit, der eine Ölmischung (Komponente a)) und einen oder mehrere Feststoffe (Komponente b)) enthält, wobei diese beiden Komponenten jeweils enthalten:
Komponente a): Ester eines Fettsäuregemischs, wobei die Fettsäuren pflanzlichen oder synthetischen Ursprungs sind und die Ölmischung enthält:
   10 - 30, vorzugsweise 12 - 25 Gew.-% Palmitinsäure,
   3 - 15, vorzugsweise 5 - 11 Gew.-% Palmitoleinsäure,
   1,5 - 15, vorzugsweise 2,5 - 10 Gew.-% Stearinsäure,
   35 - 80, vorzugsweise 40 - 75 Gew.-% Ölsäure,
   1 - 30, vorzugsweise 1,5 - 25 Gew.-% Linolsäure,
   0 - 4, vorzugsweise 0 - 2 Gew.-% Linolensäure,
Komponente b): wenigstens ein Metallhydroxid.

Die Ölmischung des erfindungsgemäßen Kits ist frei von Ölen tierischen Ursprungs, sie enthält die angegebenen Anteile langkettiger Fettsäuren, die in der Ölmischung als Ester, in der Regel als Triglyceride, Diglyceride oder Monoglyceride enthalten sind. Die genannten Gewichtsanteile beziehen sich auf den Anteil der jeweiligen Säure als solcher (ohne die Estergruppe) am Gesamtgewicht der Ölmischung. Die Ölmischung enthält ausschließlich Öle synthetischen und/oder pflanzlichen Ursprungs, bevorzugt ausschließlich Öle pflanzlichen Ursprungs.

Der Kern der Erfindung liegt in der Bereitstellung einer bestimmten Ölmischung vorzugsweise pflanzlichen Ursprungs, deren Beschaffenheit so ist, daß beim Ansetzen einer Paste oder Suspension aus dieser Ölmischung und der vorzugsweise Calciumhydroxid enthaltenden Komponente b) eine Paste bzw. Suspension entsteht, deren Konsistenz einerseits ein Einbringen in enge Wurzelkanäle sowie Zahnfleischtaschen unter im wesentlichen vollständiger Benetzbarkeit der Wandflächen dieser Zahnfleischtaschen und Kanäle und andererseits eine verzögerte Alkalitätsfreisetzung bewirkt. Diese verzögerte Alkalitätsfreisetzung entfaltet eine pulpaprophylaktische Wirkung, alkalisiert saure Entzündungsbezirke und kann die Kollagenneubildung in vivo anregen. Bei der Verwendung als Wurzelkanalfüllmaterial wird das saure Milieu in den Wurzelkanälen ins Alkalische umgestimmt, dies beseitigt Entzündungen und es bildet sich allmählich eine Hartgewebsbarriere. Die durch die Ölmischung verzögerte Alkalitätsfreisetzung vermeidet beim Aufbringen von Metallhydroxiden in wäßriger Lösung mögliche Gewebsnekrosen.

Bevorzugte Anteile der Fettsäuren in der Ölmischung sind wie folgt:
12 - 18 Gew.-% Palmitinsäure,
7 - 10 Gew.-% Palmitoleinsäure,
2,5 - 5 Gew.-% Stearinsäure,
50 - 65 Gew.-% Ölsäure,
3 - 10 Gew.-% Linolsäure,
0 - 1 Gew.-% Linolensäure.

Bevorzugt enthält die Ölmischung ausschließlich pflanzliche Öle.

Der Anteil der Ölmischung am Gesamtgewicht der Inhaltsstoffe des Kits beträgt vorzugsweise 25 - 70 Gew.-%, weiter vorzugsweise 25 - 35 Gew.-% zum Ansetzen einer Paste und 50 - 70 Gew.-%, weiter vorzugsweise 55 - 65 Gew.-%, zum Ansetzen einer dünnflüssigeren Suspension. Der entsprechende Anteil der vorzugsweise pulverförmigen Komponente b) beträgt 75 - 40 Gew.-%, weiter vorzugsweise 65 - 75 Gew.-%, weiter vorzugsweise 40 Gew.-%. Besonders bevorzugt ist ein Ölanteil von 25 Gew.-% oder höher, da aus einem entsprechenden Kit angesetzte Pasten mit einem Ölanteil von 25 Gew.-% oder höher eine Beschaffenheit aufweisen, die ein Einbringen in Wurzelkanäle dergestalt erlaubt, daß die Wandflächen dieser Wurzelkanäle im wesentlichen vollständig benetzt werden, was durch eine scharfe Abgrenzung zwischen Dentin und dem Lumen des Wurzelkanals im Röntgenbild deutlich sichtbar wird.

Die Komponente b) enthält bevorzugt Alkali- oder Erdalkalihydroxide oder Mischungen daraus, besonders bevorzugt enthält sie Calciumhydroxid.

Die genannte Aufzählung der Inhaltsstoffe des Kits bzw. einer daraus angesetzten Mischung oder Paste ist nicht abschließend, der Kit, insbesondere dessen Komponente b), kann zusätzliche Füllstoffe oder Zusatzstoffe aufweisen. Geeignet sind beispielsweise im Dentalbereich übliche Gläser oder Glaskeramiken, insbesondere Barium oder Strontiumgläser oder Ionomergläser. In der Ölmischung können zusätzlich Oxidationshemmer wie beispielsweise α-Tocopherol eingesetzt werden.

Insbesondere bei einem Kit, der zu einer Paste anmischbar ist, die als Wurzelkanalfüllung Verwendung finden soll, kann eine Röntgenopazität der Mischung erwünscht sein. Diese kann durch Zusatz einen Röntgenkontrastmittels wie beispielsweise Bariumsulfat erreicht werden. Dieses Röntgenkontrastmittel wird vorzugsweise der Komponente b) des Kits zugesetzt. Bevorzugt beträgt der Bariumsulfatanteil in dem Kit bezogen auf das Gesamtgewicht aller Komponenten wenigstens 33 Gew.-%, vorzugsweise 33 - 45 Gew.-%, weiter vorzugsweise 33 - 40 Gew.-%.

Geeignete pflanzliche Basisöle zur Herstellung der Ölmischung gemäß Komponente a) Öle enthält ausgewählt aus der Gruppe bestehend aus Olivenöl, Avocadoöl, Sonnenblumenöl, Palmöl, Palmolein und Makadamia-Nußöl. Aus diesen pflanzlichen Ölen läßt sich eine erfindungsgemäße Ölmischung ansetzen.

Aus einem erfindungsgemäßen Kit läßt sich durch Vermischen von Ölmischung und Metallhydroxidpulver eine pastöse Masse ansetzen, die ebenfalls Gegenstand der Erfindung ist. Ein erfindungsgemäßer Kit kann beispielsweise die Komponente b) bereits vorportioniert in typischen Anwendungsgrößen enthalten, so daß sich ein separates Portionieren oder Abwiegen dieser Komponente zum Herstellen einer typischerweise benötigten Pastenmenge erübrigt. Beispielsweise kann der Kit die Komponente b) portioniert in separat verpackten Teilmengen von jeweils 400 mg enthalten. Ein erfindungsgemäßer Kit kann eine oder mehrere solche Teilmengen enthalten.

Komponente a) kann ebenfalls getrennt in mehreren Teilmengen verpackt vorportioniert sein, häufig wird man jedoch die Ölmischung in einem Vorratsbehälter wie beispielsweise einer Flasche aufbewahren, aus der sie dosiert abgegeben werden kann. Beispielsweise kann auf eine vorportionierte Menge der Komponente b) von 400 mg die Ölmischung tropfenweise aufgegeben werden, ein Tropfen besitzt ein Gewicht von etwa 27 mg, so daß durch Zugabe von 5 - 7 bzw. 15 Tropfen Ölmischungsanteile von etwa 25 - 33 % bzw. 60 % eingestellt werden können. Die erfindungsgemäße pastöse Mischung eignet sich zur Verwendung als Medikament. Ein erfindungsgemäßer Kit bzw. eine daraus hergestellte pastöse Mischung eignet sich insbesondere zur Behandlung entzündlicher Zahn- oder Zahnfleischerkrankungen sowie als Wurzelkanalfüllmaterial.

Beispiele der Erfindung werden nachfolgend erläutert.

Tabelle 1 zeigt die Fettsäurezusammensetzung verschiedener pflanzlicher Öle, die als Ausgangsmaterialien zum Ansetzen einer erfindungsgemäßen Ölmischung geeignet sind.

**Tabelle 1**

| Fettsäurezusammensetzung und Peroxidzahl | | | | | | | | |
|---|---|---|---|---|---|---|---|---|
| | Monographie/ Spezifikat ion | POZ | Fettsäurezusammensetzung (%) | | | | | |
| | | | Palmitinsäure (C₁₅H₃₁COOH, ges.) | Palmitoleinsäure (C₁₅H₂₉COOH, unges.) | Stearinsäure (C₁₇H₃₃COOH, ges.) | Olsäure (C₁₇H₃₃COOH, unges.) | Linolsäure (C₁₇H₃₁COOH, 2fach | Linolensäure (C₁₇H₃₃COOH, 3 fach unges.) |
| Avocadoöl | DAC | <15 | 5-25 | 1-10 | <3 | 50-74 | 6-20 | <3 |
| Borretschsamenöl | DAC | <10 | 9-13 | <0,5 | 3-5 | 14-20 | 34-45 | 18-25 |
| Erdnussöl | EuAB | <5 | 7-16 | -- | 1,3-6,5 | 35-72 | 13-43 | <0,6 |
| Leinöl | DAC | <10 | 3-8 | <1 | 2-8 | 11-24 | 11-24 | 45-65 |
| Maiskeimöl | DAC | <10 | 8-19 | -- | 0,5-4 | 19-50 | 34-62 | <2 |
| Makadamia-Nussöl | HL* | <10 | 8-10 | 16-24 | 2-4 | 53-67 | 1,5-4 | max. 0,5 |
| Mandelöl, raff. | EuAB | <5 | 4-9 | <0,6 | <3 | 62-86 | 20-30 | <0,4 |
| Nachtkerzenöl | DAC | <10 | 4-8 | -- | 0,5-3 | 7-15 | 66-76 | 8-12 |
| Olivenöl | EuAB | <15 | 7,5-20 | <3,5 | 0,5-5 | 56-85 | 3,5-20 | <1,5 |
| Palmöl, raff. | HL* | <10 | 35-48 | max. 0,6 | 3-7 | 35-50 | 6-13 | max. 1,0 |
| Palmolein, raff. | HL* | <10 | 32-42 | -- | 3,5-5 | min. 40 | 9-14 | max. 0,8 |
| Rizinusöl | EuAB | <5 | -- | -- | -- | 5 | 2-5 | -- |
| Rüböl | DAC | <10 | 3-8 | -- | <3 | 45-65 | 18-32 | 6-14 |
| Sesamöl | EuAB | <5 | 7-12 | -- | 3,5-6 | 35-50 | 35-50 | <1 |
| Sojaöl | EuAB | <10 | 9-13 | <0,3 | 3-5 | 17-30 | 48-50 | 5-11 |
| Sonnenblumen öl | DAC | <10 | 4-9 | <1 | 1-7 | 15-35 | 50-72 | <2 |

| | | | | | | | | |
|---|---|---|---|---|---|---|---|---|
| * HL - Henry Lamoae Palmitinsäure Hexadecansäure Palmitoleinsäure 9-Hexadecensäure Ölsäure 9-Octadecensäure Linolsäure 9,12-Octadecadiensäure | | | | | | | | |

In den nachfolgenden Beispielen 1 bis 4 werden Ölmischungen (Komponente a) eines erfindungsgemäßen Kits) aus verschiedenen pflanzlichen Grundölen angesetzt. Da bei den Beispielen die pflanzlichen Grundöle aus verschiedenen Chargen stammen, ist deren Fettsäurezusammensetzung nicht bei jedem Beispiel identisch.

### Beispiel 1

| Fettsäure | | Olivenöl | | Palmolein | | Makadamia-Nußöl | | Öl-Mischung | |
|---|---|---|---|---|---|---|---|---|---|
| | | Masse | Gehalt | Masse | Gehalt | Masse | Gehalt | Masse | Gehalt |
| Palmitinsäure | C16:0 | 30 | 10,400% | 20 | 39,400% | 50 | 8,200% | 100 | 15,10% |
| Palmitoleinsäure | C16:1 | 30 | 0,600% | 20 | 0,200% | 50 | 18,000% | 100 | 9,22% |
| Stearinsäure | C18:0 | 30 | 2,500% | 20 | 4,300% | 50 | 2,800% | 100 | 3,01% |
| Ölsäure | C18:1 | 30 | 75,800% | 20 | 42,900% | 50 | 59,100% | 100 | 60,87% |
| Linolsäure | C18:2 | 30 | 8,300% | 20 | 10,900% | 50 | 3,200% | 100 | 6,27% |
| Linolensäure | C18:3 | 30 | 0,600% | 20 | 0,200% | 50 | 0,200% | 100 | 0,32% |

### Beispiel 2

| Fettsäure | | Palmöl | | Avocadoöl | | Sonnenblumenöl | | Makadamia-Nußöl | | Öl-Mischung | |
|---|---|---|---|---|---|---|---|---|---|---|---|
| | | Masse | Gehalt | Masse | Gehalt | Masse | Gehalt | Masse | Gehalt | Masse | Gehalt |
| Palmitinsäure | C16:0 | 39,56 | 43,20% | 9,89 | 13,50% | 20,07 | 6,00% | 30,38 | 8,20% | 99,9 | 22,14% |
| Palmitoleinsäure | C16:1 | 39,56 | 0,20% | 9,89 | 5,10% | 20,07 | 0,10% | 30,38 | 18,00% | 99,9 | 6,08% |
| Stearinsäure | C18:0 | 39,56 | 4,70% | 9,89 | 0,90% | 20,07 | 3,70% | 30,38 | 2,80% | 99,9 | 3,55% |
| Ölsäure | C18:1 | 39,56 | 39,40% | 9,89 | 63,10% | 20,07 | 23,60% | 30,38 | 59,10% | 99,9 | 44,56% |
| Linolsäure | C18:2 | 39,56 | 10,40% | 9,89 | 15,60% | 20,07 | 64,70% | 30,38 | 3,20% | 99,9 | 19,63% |
| Linolensäure | C18:3 | 39,56 | 0,20% | 9,89 | 1,00% | 20,07 | 0,40% | 30,38 | 0,20% | 99,9 | 0,32% |

### Beispiel 3

| Fettsäure | | Olivenöl | | Palmöl | | Makadamia-Nußöl | | Öl-Mischung | |
|---|---|---|---|---|---|---|---|---|---|
| | | Masse | Gehalt | Masse | Gehalt | Masse | Gehalt | Masse | Gehalt |
| Palmitinsäure | C16:0 | 30 | 10,400% | 20 | 43,200% | 50 | 8,200% | 100 | 15,86% |
| Palmitoleinsäure | C16:1 | 30 | 0,600% | 20 | 0,200% | 50 | 18,000% | 100 | 9,22% |
| Stearinsäure | C18:0 | 30 | 2,500% | 20 | 4,700% | 50 | 2,800% | 100 | 3,09% |
| Ölsäure | C18:1 | 30 | 75,800% | 20 | 39,400% | 50 | 59,100% | 100 | 60,17% |
| Linolsäure | C18:2 | 30 | 8,300% | 20 | 10,400% | 50 | 3,200% | 100 | 6,17% |
| Linolensäure | C18:3 | 30 | 0,600% | 20 | 0,200% | 50 | 0,200% | 100 | 0,32% |

### Beispiel 4

| Fettsäure | | Palmöl | | Avocadoöl | | Sonnenblumenöl | | Makadamia-Nußöl | | Öl-Mischung | |
|---|---|---|---|---|---|---|---|---|---|---|---|
| | | Masse | Gehalt | Masse | Gehalt | Masse | Gehalt | Masse | Gehalt | Masse | Gehalt |
| Palmitinsäure | C16:0 | 20 | 43,20% | 20 | 13,50% | 20 | 6,00% | 40 | 8,20% | 100 | 15,82% |
| Palmitoleinsäure | C16:1 | 20 | 0,20% | 20 | 5,10% | 20 | 0,10% | 40 | 18,00% | 100 | 8,28% |
| Stearinsäure | C18:0 | 20 | 4,70% | 20 | 0,90% | 20 | 3,70% | 40 | 2,80% | 100 | 2,98% |
| Ölsäure | C18:1 | 20 | 39,40% | 20 | 63,10% | 20 | 23,60% | 40 | 59,10% | 100 | 48,86% |
| Linolsäure | C18:2 | 20 | 10,40% | 20 | 15,60% | 20 | 64,70% | 40 | 3,20% | 100 | 19,42% |
| Linolensäure | C18:3 | 20 | 0,20% | 20 | 1,00% | 20 | 0,40% | 40 | 0,20% | 100 | 0,40% |

### Beispiel 5

Die kinematische Viskosität der Ölmischungen gemäß Beispielen 2 und 3 wurde bestimmt mit einem Viskosimeter nach Ubellohde (dreischenklig S4 lauda) bei verschiedenen Temperaturen. Die Ergebnisse sind in Tabelle 2 angegeben (jeweils Mittelwerte aus drei Messungen).

**Tabelle 2**

| Temperatur | | Beispiel 2 | Beispiel 3 |
|---|---|---|---|
| 20,0°C: | 1. Bestimmung | 86,86 mm²/s | 89,60 mm²/s |
| | (MW aus 3 Messungen) | | |
| | | | |
| | 2. Bestimmung | 87,25 mm² /s | 89,76 mm²/s |
| | (MW aus 3 Messungen) | | |
| 25,0°C: | 1. Bestimmung | 69,51 mm²/s | 71,64 mm²/s |
| | (MW aus 3 Messungen) | | |
| | | | |
| | 2. Bestimmung | 69,52 mm²/s | 71,55 mm²/s |
| | (MW aus 3 Messungen) | | |
| 37,0°C: | 1. Bestimmung | 42,69 mm²/s | 44,33 mm²/s |
| | (MW aus 3 Messungen) | | |
| | | | |
| | 2. Bestimmung (MW aus 3 Messungen) | 42,71 mm²/s | 44,34 mm²/s |

Diese Viskositäten der Ölmischungen eignen sich zum Ansetzen von Pasten, die sich als Wurzelkanalfüllungen auch in enge Wurzelkanäle einbringen lassen.

### Beispiel 6: Rezeptur einer dünnflüssigen Suspension für Zahnbetterkrankungen

250 mg Ca(OH)₂ und 400 mg der Ölmischung aus Beispiel 2 oder Beispiel 4 werden unmittelbar vor der Anwendung zu einer Suspension verrührt. Die Suspension eignet sich zur Behandlung von Zahnbetterkrankungen und kann aufgrund ihrer geringen Viskosität auch in Zahnfleischtaschen eingebracht werden.

### Beispiel 7: Rezepturen medikamentöser Wurzelkanaleinlagen

400 mg Ca(OH)₂ und 136 mg der Ölmischung aus Beispiel 3 werden unmittelbar vor dem Gebrauch vermischt. Die Mischung wird als entzündungshemmende Wurzelkanaleinlage verwendet.

Sofern eine etwas dünnflüssigere Konsistenz gewünscht wird, werden 400 mg Ca(OH)₂ mit 190 mg der Ölmischung aus Beispiel 3 oder Beispiel 4 angesetzt.

### Beispiel 8: Rezeptur eines Wurzelkanalfüllmaterials

200 mg Ca(OH)₂ und 200 mg BaSO₄ werden unmittelbar vor dem Gebrauch mit 136 mg der Ölmischung aus Beispiel 1 vermischt. Man erhält ein Wurzelkanalfüllmaterial, das aufgrund seines Bariumsulfatgehaltes von etwas über 37 Gew.-% eine gute Röntgenopazität aufweist.

## Patentansprüche

1. Kit, der eine Ölmischung (Komponente a)) und einen Feststoff (Komponente b)) enthält, **dadurch gekennzeichnet, dass** diese beiden Komponenten enthalten:
Komponente a): Ester eines Fettsäuregemischs, wobei die Fettsäuren pflanzlichen oder synthetischen Ursprungs sind und die Ölmischung enthält:
10- 30 Gew.-% Palmitinsäure,
3-15 Gew.-% Palmitoleinsäure,
1,5 - 15 Gew.-% Stearinsäure,
35 - 80 Gew.-% Ölsäure,
1-30 Gew.-% Linolsäure,
0 - 4 Gew.-% Linolensäure,
Komponente b): wenigstens ein Metallhydroxid.

2. Kit nach Anspruch 1, **dadurch gekennzeichnet, dass** die Ölmischung 12 - 25 Gew.-% Palmitinsäure enthält.

3. Kit nach Anspruch 1 oder 2, **dadurch gekennzeichnet, dass** die Ölmischung 5 - 11 Gew.-% Palmitoleinsäure enthält.

4. Kit nach einem der Ansprüche 1 bis 3, **dadurch gekennzeichnet, dass** die Ölmischung 2,5 - 10 Gew.-% Stearinsäure enthält.

5. Kit nach einem der Ansprüche 1 bis 4, **dadurch gekennzeichnet, dass** die Ölmischung 40 - 75 Gew.-% Ölsäure enthält.

6. Kit nach einem der Ansprüche 1 bis 5, **dadurch gekennzeichnet, dass** die Ölmischung 1,5 - 25 Gew.-% Linolsäure enthält.

7. Kit nach einem der Ansprüche 1 bis 6, **dadurch gekennzeichnet, dass** die Ölmischung 0-2 Gew.-% Linolensäure enthält.

8. Kit nach einem der Ansprüche 1 bis 7, **dadurch gekennzeichnet, dass** die Ölmischung enthält:
12 - 18 Gew.-% Palmitinsäure,
7 - 10 Gew.-% Palmitoleinsäure,
2,5 - 5 Gew.-% Stearinsäure,
50 - 65 Gew.-% Ölsäure,
3-10 Gew.-% Linolsäure,
0 - 1 Gew.-% Linolensäure.

9. Kit nach einem der Ansprüche 1 bis 8, **dadurch gekennzeichnet, dass** die Ölmischung (Komponente a)) ausschließlich pflanzliche Öle enthält.

10. Kit nach einem der Ansprüche 1 bis 9, **dadurch gekennzeichnet, dass** der Anteil der Komponente a) am Gesamtgewicht der Inhaltsstoffe des Kits 15 - 70 Gew.-%, vorzugsweise 25 - 35 Gew.-%, und der entsprechende Anteil der Komponente b) 60 - 85 Gew.-%, vorzugsweise 65 - 75 Gew.-%, beträgt.

11. Kit nach einem der Ansprüche 1 bis 9, **dadurch gekennzeichnet, dass** der Anteil der Komponente a) am Gesamtgewicht der Inhaltsstoffe des Kits 50 - 70 Gew.-%, vorzugsweise 55 - 65 Gew.-%, und der entsprechende Anteil der Komponente b) 30 - 50 Gew.-%, vorzugsweise 45 - 55 Gew.-%, beträgt.

12. Kit nach einem der Ansprüche 1 bis 11, **dadurch gekennzeichnet, dass** die Komponente b) Alkali- oder Erdalkalihydroxide enthält.

13. Kit nach Anspruch 12, **dadurch gekennzeichnet, dass** die Komponente b) Calciumhydroxid enthält.

14. Kit nach einem der Ansprüche 1 bis 13, **dadurch gekennzeichnet, dass** die Komponente b) zusätzlich ein Röntgenkontrastmittel enthält.

15. Kit nach Anspruch 14, **dadurch gekennzeichnet, dass** das Röntgenkontrastmittel Bariumsulfat enthält.

16. Kit nach Anspruch 15, **dadurch gekennzeichnet, dass** der Bariumsulfatanteil in dem Kit bezogen auf das Gesamtgewicht aller Komponenten wenigstens 33 Gew.-%, vorzugsweise 33 - 45 Gew.-%, weiter vorzugsweise 33 - 40 Gew.-% beträgt.

17. Kit nach einem der Ansprüche 1 bis 16, **dadurch gekennzeichnet, dass** die Ölmischung (Komponente a)) Öle enthält ausgewählt aus der Gruppe bestehend aus Olivenöl, Avocadoöl, Sonnenblumenöl, Palmöl, Palmolein, und Makadamia-Nußöl.

18. Pastöse Mischung, enthaltend die Komponenten a) und b) eines Kits nach einem der Ansprüche 1 bis 17.

19. Pastöse Mischung nach Anspruch 18 zur Verwendung als Medikament.

20. Verwendung eines Kits nach einem der Ansprüche 1 bis 17 zur Herstellung eines Medikaments zur Behandlung entzündlicher Zahnbett- oder Zahnfleischerkrankungen.

21. Verwendung eines Kits nach einem der Ansprüche 1 bis 17 zur Herstellung eines Wurzelkanalfüllmaterials.

## Claims

1. Kit which contains an oil mixture (component a)) and a solid (component b)), **characterized in that** these two components contain:
component a): esters of a fatty acid mixture, wherein the fatty acids are of vegetable or synthetic origin and the oil mixture contains:
10-30% by weight palmitic acid,
3-15% by weight palmitoleic acid,
1.5-15% by weight stearic acid,
35-80% by weight oleic acid,
1-30% by weight linoleic acid,
0-4% by weight linolenic acid,
component b): at least one metal hydroxide.

2. Kit according to Claim 1, **characterized in that** the oil mixture contains 12-25% by weight palmitic acid.

3. Kit according to Claim 1 or 2, **characterized in that** the oil mixture contains 5-11% by weight palmitoleic acid.

4. Kit according to one of Claims 1 to 3, **characterized in that** the oil mixture contains 2.5-10% by weight stearic acid.

5. Kit according to one of Claims 1 to 4, **characterized in that** the oil mixture contains 40-75% by weight oleic acid.

6. Kit according to one of Claims 1 to 5, **characterized in that** the oil mixture contains 1.5-25% by weight linoleic acid.

7. Kit according to one of Claims 1 to 6, **characterized in that** the oil mixture contains 0-2% by weight linolenic acid.

8. Kit according to one of Claims 1 to 7, **characterized in that** the oil mixture contains:
12-18% by weight palmitic acid,
7-10% by weight palmitoleic acid,
2.5-5% by weight stearic acid,
50-65% by weight oleic acid,
3-10% by weight linoleic acid,
0-1% by weight linolenic acid.

9. Kit according to one of Claims 1 to 8,
**characterized in that** the oil mixture (component a)) contains exclusively vegetable oils.

10. Kit according to one of Claims 1 to 9, **characterized in that** the fraction of component a) of the total weight of the components of the kit is 15-70% by weight, preferably 25-35% by weight, and the corresponding fraction of component b) is 60-85% by weight, preferably 65-75% by weight.

11. Kit according to one of Claims 1 to 9, **characterized in that** the fraction of component a) of the total weight of the components of the kit is 50-70% by weight, preferably 55-65% by weight, and the corresponding fraction of component b) is 30-50% by weight, preferably 45-55% by weight.

12. Kit according to one of Claims 1 to 11, **characterized in that** the component b) contains alkali metal hydroxides or alkaline earth metal hydroxides.

13. Kit according to Claim 12, **characterized in that** the component b) contains calcium hydroxide.

14. Kit according to one of Claims 1 to 13, **characterized in that** the component b) additionally contains an X-ray contrast material.

15. Kit according to Claim 14, **characterized in that** the X-ray contrast material contains barium sulphate.

16. Kit according to Claim 15, **characterized in that** the barium sulphate fraction in the kit, based on the total weight of all components, is at least 33% by weight, preferably 33-45% by weight, further preferably 33-40% by weight.

17. Kit according to one of Claims 1 to 16, **characterized in that** the oil mixture (component a)) contains oils selected from the group consisting of olive oil, avocado oil, sunflower oil, palm oil, palmolein, and macadamia nut oil.

18. Pasty mixture containing the components a) and b) of a kit according to one of Claims 1 to 17.

19. Pasty mixture according to Claim 18 for use as medicament.

20. Use of a kit according to one of Claims 1 to 17 for producing a medicament for treating inflammatory tooth socket or gum diseases.

21. Use of a kit according to one of Claims 1 to 17 for producing a root canal filler.

## Revendications

1. Préparation, contenant un mélange huileux (composant a)) et un solide (composant b)), **caractérisée en ce que** les deux composants comprennent :
composant a) : ester d'un mélange d'acides gras, lesdits acides gras étant d'origine végétale ou synthétique et le mélange huileux contenant :
de 10 à 30 % en poids d'acide palmitique,
de 3 à 15 % en poids d'acide palmitoléique,
de 1,5 à 15 % en poids d'acide stéarique,
de 35 à 80 % en poids d'acide oléique,
de 1 à 30 % en poids d'acide linoléique,
de 0 à 4 % en poids d'acide linolénique,
composant b) : au moins un hydroxyde métallique.

2. Préparation selon la revendication 1, **caractérisée en ce que** le mélange huileux contient de 12 à 25 % en poids d'acide palmitique.

3. Préparation selon la revendication 1 ou 2, **caractérisée en ce que** le mélange huileux contient de 5 à 11 % en poids d'acide palmitoléique.

4. Préparation selon l'une des revendications 1 à 3, **caractérisée en ce que** le mélange huileux contient de 2,5 à 10 % en poids d'acide stéarique.

5. Préparation selon l'une des revendications 1 à 4, **caractérisée en ce que** le mélange huileux contient de 40 à 75 % en poids d'acide oléique.

6. Préparation selon l'une des revendications 1 à 5, **caractérisée en ce que** le mélange huileux contient de 1,5 à 25 % en poids d'acide linoléique.

7. Préparation selon l'une des revendications 1 à 6, **caractérisée en ce que** le mélange huileux contient de 0 à 2 % en poids d'acide linolénique.

8. Préparation selon l'une des revendications 1 à 7, **caractérisée en ce que** le mélange huileux contient :
de 12 à 18 % en poids d'acide palmitique,
de 7 à 10 % en poids d'acide palmitoléique,
de 2,5 à 5 % en poids d'acide stéarique,
de 50 à 65 % en poids d'acide oléique,
de 3 à 10 % en poids d'acide linoléique,
de 0 à 1 % en poids d'acide linolénique.

9. Préparation selon l'une des revendications 1 à 8, **caractérisée en ce que** le mélange huileux (composant a)) contient exclusivement des huiles végétales.

10. Préparation selon l'une des revendications 1 à 9, **caractérisée en ce que** la part du composant a) dans le poids total des composants de la préparation est comprise entre 15 et 70 %, de préférence entre 25 et 35 %, et **en ce que** la part en poids correspondante du composant b) est comprise entre 60 et 85 %, de préférence entre 65 et 75 %.

11. Préparation selon l'une des revendications 1 à 9, **caractérisée en ce que** la part du composant a) dans le poids total des composants de la préparation est comprise entre 50 et 70 %, de préférence entre 55 et 65%, et **en ce que** la part en poids correspondante du composant b) est comprise entre 30 et 50 %, de préférence entre 45 et 55 %.

12. Préparation selon l'une des revendications 1 à 11, **caractérisée en ce que** le composant b) contient des hydroxydes alcalins ou alcalino-terreux.

13. Préparation selon la revendication 12, **caractérisée en ce que** le composant b) contient de l'hydroxyde de calcium.

14. Préparation selon l'une des revendications 1 à 13, **caractérisée en ce que** le composant b) contient en outre une substance de contraste radiologique.

15. Préparation selon la revendication 14, **caractérisée en ce que** la substance de contraste radiologique contient du sulfate de baryum.

16. Préparation selon la revendication 15, **caractérisée en ce que** la part de sulfate de baryum dans la préparation est d'au moins 33 % du poids total de l'ensemble des composants, et est de préférence comprise entre 33 et 45 % en poids, et notamment entre 33 et 40 % en poids.

17. Préparation selon l'une des revendications 1 à 16, **caractérisée en ce que** le mélange huileux (composant a)) contient des huiles sélectionnées dans le groupe formé par l'huile d'olive, l'huile d'avocat, l'huile de tournesol, l'huile de palme, l'acide oléique de palme, et l'huile de noix de macadamia.

18. Mélange pâteux, contenant les composants a) et b) d'une préparation selon l'une des revendications 1 à 17.

19. Mélange pâteux selon la revendication 18, destiné à être utilisé comme médicament.

20. Utilisation d'un mélange selon l'une des revendications 1 à 17 pour la fabrication d'un médicament pour le traitement d'affections inflammatoires des alvéoles dentaires ou des gencives.

21. Utilisation d'un mélange selon l'une des revendications 1 à 17 pour la fabrication d'une matière d'obturation des canaux dentaires.
